(19) Europäisches Patentamt

European Patent Office

Office européen des brevets

(11) **EP 1 264 899 A2**

(12) **EUROPEAN PATENT APPLICATION**

(43) Date of publication:
**11.12.2002 Bulletin 2002/50**

(51) Int Cl.⁷: **C12Q 1/68**

(21) Application number: **02012176.0**

(22) Date of filing: **03.06.2002**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | (72) Inventors:<br>• **Hayashizaki, Yoshihide**<br>**Tukuba-shi, Ibaraki 305-0061 (JP)**<br>• **Okazaki, Yasushi, c/o Riken Yokohama Institute**<br>**Yokohama-shi, Kanagawa 230-0045 (JP)** |
| (30) Priority: **04.06.2001 JP 2001167910** | (74) Representative: **Gervasi, Gemma, Dr.**<br>**Notarbartolo & Gervasi S.p.A.,**<br>**Corso di Porta Vittoria, 9**<br>**20122 Milano (IT)** |
| (71) Applicants:<br>• **Riken**<br>**Wako-shi, Saitama 351-0198 (JP)**<br>• **Hayashizaki, Yoshihide**<br>**Tsukuba-shi, Ibaraki 305-0061 (JP)** | |

(54) **Methods and kits for preparing and detecting RNA probes**

(57) A method of preparing labeled RNA probe by reacting RNA polymerase in the presence of a DNA fragment comprising a promoter sequence for the RNA polymerase and substrates of the RNA polymerase. In the method, at least one of said substrates comprises said label, and said RNA polymerase is mutant RNA polymerase where at least one of the amino acids of wild type RNA polymerase has been modified to permit incorporation of the substrate having a label or to improve the incorporation of the substrate having a label. A method of detecting targeted nucleic acid in which targeted nucleic acid and labeled RNA probe prepared by the above method are mixed and RNA probe that has hybridized with the targeted nucleic acid is selectively detected. A kit for preparing labeled RNA probe comprising (1) RNA polymerase, (2) DNA comprising a promoter sequence for said RNA polymerase, (3) substrates of said RNA polymerase, and (4) optionally an instruction manual. In the kit, at least one of said substrates comprises a label and said RNA polymerase is mutant RNA polymerase where at least one of the amino acids of wild type RNA polymerase has been modified to permit incorporation of said substrate having a label or to improve the incorporation of said substrate having a label.

EP 1 264 899 A2

**Description**

**Technical Field**

The present invention relates to a method of preparing RNA probe, a method of detecting targeted nucleic acid, and a kit for preparing RNA probe.

**Technical Background**

Nucleic acid probes are employed in gene diagnosis, specification of pathogenic bacteria, detection of single nucleic acid polymorphisms, and detection of certain nucleic acids (targeted nucleic acids). The nucleic acid probe is mixed with the targeted nucleic acid and the presence or absence of hybridization of the nucleic acid probe and the targeted nucleic acid is detected, for example, by means of a label such as a fluorescent label present on the nucleic acid probe.

Since nucleic acid probes are readily synthesized by DNA synthesizers, DNA probes are primarily employed. Further, fluorescent labels are often employed for ease of detecting nucleic acid probe that has hybridized with the targeted nucleic acid, however also non-fluorescent labels, such as RI may be employed

DNA microarrays and DNA chips in which numerous targeted nucleic acids are fixed to a substrate have been developed in recent years. The appearance of an easily handled technique for detecting targeted nucleic acid using nucleic acid probe having high detection sensitivity is being awaited.

This technique for detecting targeted nucleic acid using nucleic acid probe will have to be able to detect just the nucleic acid probe that has hybridized with a targeted nucleic acid in the presence of nucleic acid probe that has not hybridized. One method of detecting just nucleic acid probe that has hybridized with a targeted nucleic acid is known. In this method, the nucleic acid probe and the targeted nucleic acid are respectively labeled with a fluorescent label having different excitation wavelength and light-emission wavelength and in which the excitation wavelength of the one duplicates the light-emission wavelength of the other. If a laser beam of a wavelength exciting just one of the fluorescent labels is applied after hybridization, the excitation energy is transferred to the other fluorescent label, causing the other fluorescent label to emit light and thus permitting detection of just the nucleic acid probe that has hybridized with the targeted nucleic acid. However, this method has the drawback that it is necessary to attach a fluorescent label to the targeted nucleic acid, which is a tedious operation.

A method of eliminating this drawback is to divide a single nucleic probe into two, attach two fluorescent labels of the same combination as above to each of the divided probes, and ensure that only when both nucleic probes have hybridized is the prescribed fluorescent light obtained. However, this method is problematic in that two probes must ultimately be prepared.

Currently, fluorescent-labeled probes incorporating cyanine 3-dUTP and cyanine 5-dUTP are often employed in the detection of targeted DNA employing a DNA microarray. In this process, a reverse transcription reaction employing random primer has been disclosed as a method of preparing probe to somewhat enhance the signal intensity (Okazaki, Y., et al., Expression profile analysis employing mouse cDNA microarray (Cell Technology, Vol. 18, Number 6, 1999)).

In methods of detecting targeted nucleic acid using nucleic acid probe, particularly in methods employing DNA chips or DNA microarrays, high detection sensitivity (a high S/N ratio) is desirable. That is because in DNA chips and DNA arrays, the presence or absence of hybridization must be detected from a signal (for example, light emission) from single molecules of nucleic acid probe hybridized to single molecules of targeted nucleic acid. Increasing the absolute quantity of signal from the nucleic acid probe effectively heightens the S/N ratio of the signal from the nucleic acid probe.

All of the methods set forth above employ DNA probe. However, a method of detecting targeted amino acids using florescent-labeled RNA is also known (Hughes, T. R., et al., Nature Biotechnol. 19 (2001) 342-247). This method of detecting a targeted amino acid with an RNA probe has the advantage over methods of detecting targeted nucleic acid with DNA probe of permitting the elimination of unhybridized probe using RNase or the like. Since RNA/DNA has greater hybridization stringency than DNA/DNA, a high S/N ratio signal is achieved, and the method of detecting a targeted nucleic acid employing RNA probe has a further advantage over DNA probe in that a clear, stable signal is constantly obtained. However, this fluorescent-labeled RNA probe is prepared by adding a fluorescent label by a two-step chemosynthesis process to RNA probe obtained by transcription employing RNA polymerase with a cDNA template.

The operation of adding a fluorescent label by chemosynthesis is, in the end, a problematic additional step in view of the stability of RNA. Ideally, it would be possible to directly obtain fluorescent-labeled RNA probe by transcription with RNA polymerase. Fluorescent-labeled ribonucleotide is already available as a reagent, and the present inventors thought that fluorescent-labeled RNA probe could be directly obtained by transcription with RNA polymerase using this fluorescent-labeled ribonucleotide as part of the substrate. However, despite attempts at transcription with RNA probe with T7 RNA polymerase using fluorescent-labeled ribonucleotide in the form of cyanine 3-UTP and cyanine 5-UTP, the present inventors were unable to obtain fluorescent-labeled RNA probe (see the data provided in the examples set forth below). This was thought to have resulted because RNA polymerase did not recognize fluorescent-labeled ribonucleotide such as cyanine 3-UTP and cyanine 5-UTP as substrate, and thus did not incorporate it into the RNA chain.

Accordingly, an object of the present invention is to provide a method of preparing, by transcription reaction employing RNA polymerase, a labeled RNA probe which comprises a fluorescent label such as cyanine 3-UTP or cyanine 5-UTP and which ensure yielding a high S/N ratio in the detection of nucleic acid by hybridization with a target nucleic acid. A further object of the present invention is to provide a kit for preparing RNA labeled RNA probe.

A still further object of the present invention is to provide a method of detecting targeted nucleic acid using the labeled RNA probe obtained by the above-described method.

**Summary of the Invention**

The present invention relates to a method of preparing labeled RNA probe by reacting RNA polymerase in the presence of a DNA fragment comprising a promoter sequence for said RNA polymerase and substrates of said RNA polymerase, characterized in that

at least one of said substrates comprises said label, and

said RNA polymerase is mutant RNA polymerase where at least one of the amino acids of wild type RNA polymerase has been modified to permit incorporation of the substrate having a label or to improve the incorporation of the substrate having a label.

In the above invention,

said substrates are preferably ribonucleotide 5' triphosphates comprising ATP, GTP, CTP, and UTP, or derivatives thereof (referred to hereinafter as NTP derivatives), and part or all of one or more of these NTP derivatives comprises said label;

said label is preferably a fluorescent label such as cyanine 3 or cyanine 5;

said mutant RNA polymerase is preferably RNA polymerase obtained by substitution, insertion, or deletion of at least one amino acid present at a nucleotide bonding site of wild type RNA polymerase;

said mutant RNA polymerase is preferably RNA polymerase obtained by substituting tyrosine for at least one amino acid present at a nucleotide bonding site of wild type RNA polymerase, in which the amino acid substituted may be phenylalanine;

the amino acid present at a nucleotide bonding site is preferably an amino acid in the loop between helix Y and helix Z and/or an amino acid in the loop between helix Z and helix AA;

the mutant RNA polymerase is preferably from T7 phage, T3 phage, SP6 phage, or K11 phage;

the mutant RNA polymerase is preferably wild type RNA polymerase in which at least one of the amino acids in a region corresponding to amino acid residues 641-667 of RNA polymerase from T7 phage is modified;

the mutant RNA polymerase is preferably RNA polymerase from T7 phage having tyrosine as amino acid residue 644 or 667.

the mutant RNA polymerase is RNA polymerase in which tyrosine is substituted for the number 644 amino acid residue phenylalanine of wild type T7 RNA polymerase and proline may be further substituted for the number 665 amino acid residue leucine of wild type T7 RNA polymerase;

the mutant RNA polymerase is RNA polymerase in which tyrosine is substituted for the number 667 amino acid residue phenylalanine of wild type T7 RNA polymerase and proline may be further substituted for the number 665 amino acid residue leucine of wild type T7 RNA polymerase;

the mutant polymerase is preferably RNA polymerase in which tyrosine is substituted for the number 644 amino acid residue phenylalanine and tyrosine is substituted for the number 667 amino acid residue phenylalanine of wild type T7 RNA polymerase and proline may be further substituted for the number 665 amino acid residue leucine of wild type T7 RNA polymerase;

the mutant RNA polymerase is preferably RNA polymerase from T3 phage having tyrosine at the number 645 or 668 amino acid residue;

the mutant RNA polymerase is preferably RNA polymerase from K11 phage having tyrosine between the number 663-668 amino acid residues, or at the number 690 amino acid residue; and

the mutant RNA polymerase is RNA polymerase from SP6 phage having tyrosine between the number 633-638 amino acid residues, or at the number 670 amino acid residue.

The present invention further relates to a method of detecting targeted nucleic acid in which targeted nucleic acid and labeled RNA probe prepared by the method according to the above-mentioned present invention are mixed and RNA probe that has hybridized with the targeted nucleic acid is selectively detected.

In the method of detection,

following the mixing and hybridization, the mixture is preferably treated with RNase and the remaining targeted nucleic acid and the hybrid with RNA probe are detected to conduct the selective detection;

the targeted nucleic acid is preferably fixed to a substrate;

the targeted nucleic acid is preferably DNA, peptide nucleic acid, or RNA; and

said targeted nucleic acid is preferably in the form of an oligonucleotide array or cDNA microarray.

The present invention still further relates to a kit for preparing labeled RNA probe comprising

(1) RNA polymerase,

(2) DNA comprising a promoter sequence for said RNA polymerase,

(3) substrates of said RNA polymerase, and

(4) optionally an instruction manual;

characterized in that at least one of said substrates comprises a label and

said RNA polymerase is mutant RNA polymerase where at least one of the amino acids of wild type RNA polymerase has been modified to permit incorporation of said substrate having a label or to improve the incorporation of said substrate having a label.

The kit may further comprises a means of linking the DNA comprising a promoter sequence and the template DNA for preparing probe in which the means of linking the DNA comprising a promoter sequence and the template DNA for preparing probe may be DNA polymerase, or DNA polymerase and reverse transcriptase.

In the kit, said substrates preferably comprises all or some from among ribonucleotide 5' triphosphates consisting of ATP, GTP, CTP, and UTP, or derivatives thereof (referred to hereinafter as NTP derivatives), and in addition to said NTP derivatives, at least one NTP derivative all or part of which has labels;

said kit preferably comprises two or more NTP derivatives all or part of which have labels;

said label is preferably a fluorescent label; and

said fluorescent label is preferably cyanine 3 or cyanine 5.

## Brief Description of the Figures

Fig. 1 is a photograph of an electrophoretic gel in which RNA prepared with mutant RNA polymerase has been dyed with EtBr.

Fig. 2 is a photograph of an electrophoretic gel in which RNA prepared with wild type RNA polymerase has been dyed with EtBr.

Fig. 3 is a microarray pattern obtained in Example 2 with labeled RNA probe prepared with mutant RNA polymerase.

## Definition of Terms

(1) RNA probe

"RNA probe" means RNA that is caused to hybridize with a targeted nucleic acid. The term RNA probe includes RNA hybridizing with targeted DNA in the form of an oligonucleotide array, cDNA microarray, or the like.

(2) Targeted DNA

"Targeted DNA" means DNA that is caused to hybridize with probe. This includes DNA that is fixed to a substrate and caused to hybridize with RNA probe. Targeted DNA may be in the form of a polynucleotide array or cDNA microarray.

(3) Oligonucleotide array

"Oligonucleotide array" means oligonucleotides that are densely formed by chemosynthesis on a substrate such as slide glass.

(4) cDNA microarray

"cDNA microarray" means a cDNA library amplified by PCR that is fixed on a substrate such as slide glass.

Method of Preparing RNA Probe

In the method of preparing RNA probe of the present invention, RNA polymerase is reacted in the presence of a DNA fragment comprising a promoter sequence for said RNA polymerase and substrates of said RNA polymerase to prepare labeled RNA probe. However, at least one of the substrates comprises a label and the RNA polymerase is mutant RNA polymerase in which at least one amino acid of wild type RNA polymerase has been modified to permit incorporation of the substrate having the label or to improve incorporation of the substrate having the label.

The RNA probe referred to in the present invention is an RNA fragment capable of hybridizing with the targeted nucleic acid under normal nucleic acid hybridization conditions (for example, the conditions employed in the Southern blotting method or Northern blotting method). The number of bases or the sequence (arrangement or sequence of bases) of the RNA probe of the present invention is not specifically limited. However, the probe is a labeled RNA fragment capable of hybridizing with the targeted nucleic acid under the above-specified conditions.

(Labeled substrates)

Examples of labeled substrates are fluorescent substrates, chemoluminescent substrates, radioactive isotope elements (RI), and stable isotope elements. Examples of fluorescent substrates are pyrene, coumarin, diethylaminocoumarin, fluorescein chlorotriazinyl, fluorescein, 5-FAM (5-carboxyflorescein), eosin, 6-JOE (6-carboxy-4',5'-dichloro-2',7'-dimethoxyfluorescein), R6G (rhodamine 6G), tetramethylrhodamine, 5-TAMRA (5-carboxytetramethylrhodamine), R110 (rhodamine 110), lissamine, 5-ROX (5-carboxy-X-rhodamine), naphthofluorescein, Texas red, phycoerythrin, rodamin, cyanine 2, cyanine 3, cyanine 3.5, cyanine 5, cyanine 5.5, cyanine 7, FluorX, and 4,4-difluoro-5,7-dimethyl-4-bora-3a,4a-diaza-s-indacene-3-propionic acid (BODIPY FL). In the method of detecting target nucleic acids described further below, probes having two or more fluorescent labels may be employed. However, each label has a fluorescent color of the fluorescent label clearly distinguished from the others.

Further, examples of readily available labeled RNA polymerase substrates are RNA polymerase substrates labeled with fluorescein, coumarin, tetramethylrhodamine, Texas red, lissamine, naphthofluorescein, fluorescein chlorotriazinyl, pyrene, cyanine 3, and cyanine 5. These are available as commercial products (for example, from NEN™ Life Science Products, Inc.)

(Mutant RNA polymerase)

The mutant RNA polymerase employed in the method of preparing RNA probe of the present invention is wild type RNA polymerase in which at least one amino acid has been modified to incorporate the above-described labeled substrate. Mutant RNA polymerases will be described in detail below.

A known mutant RNA polymerase is described in Japanese Patent Un-examined Publication No. Hei 11-75867 and US Patent No. 6,365,350. That mutant RNA polymerase comprises wild type RNA polymerase in which at least one amino acid has been modified to increase the ability to incorporate 3'-deoxyribonucleotides or their derivatives relative to the ability of the corresponding wild type RNA polymerase, and was developed primarily for use in methods of sequencing DNA terminated by 3'-deoxyribonucleotides or their derivatives. It is known that 3'-deoxyribonucleotides and their derivatives are recognized as substrate by wild type RNA polymerase and can be employed in the synthesis of RNA. However, incorporation efficiency is poor, and the above-described mutant RNA polymerase affords improvement in this regard.

By contrast, the present inventors searched for RNA polymerase capable of incorporating substrate in the form of labeled substrate (label NTP (NTP=ATP, GTP, CTP, UTP)) that was not incorporated into the RNA strand as substrate by wild type RNA polymerase, resulting in the discovery that the mutant RNA polymerase described in the above mentioned Japanese Patent Un-examined Publication No. Hei 11-75867 and US Patent No. 6,365,350 satisfied this condition.

That is, the mutant RNA polymerase described in Patent Application Publication Number Hei 11-75867 and US Patent No. 6,365,350 can be employed as the mutant RNA polymerase employed in the present invention.

More specifically, the mutant RNA polymerase can be RNA polymerase obtained by substituting, inserting, or deleting at least one amino acid present at a nucleotide bonding site in wild type RNA polymerase.

Further, the mutant RNA polymerase can be RNA polymerase obtained by substituting tyrosine for at least one amino acid present at a nucleotide bonding site in wild type RNA polymerase. More specifically, the amino acid that is replaced by substitution can be phenylalanine.

The amino acid present at a nucleotide bonding site can be an amino acid in the loop between helix Y and helix Z and/or an amino acid present in the loop between helix Z and helix AA.

The mutant RNA polymerase may be prepared from T7 phage, T3 phage, SP6 phage, or K11 phage.

More specifically, the mutant RNA polymerase may be wild type RNA polymerase in which at least one amino acid in the region corresponding to amino acid residues 641-667 in RNA polymerase derived from T7 phage has been modified. More specifically, the mutant RNA polymerase may be RNA polymerase that is from T7 phage and has tyrosine at amino acid residue number 644 and/or 667; RNA polymerase obtained by substituting tyrosine for the number 644 amino acid residue phenylalanine in wild type T7RNA polymerase, or RNA polymerase obtained by substituting tyrosine for the number 667 amino acid residue phenylalanine in wild type T7 RNA polymerase. These wild type T7 RNA polymerases may also be RNA polymerases in which proline is substituted for the number 665 amino acid residue leucine.

Further, the mutant RNA polymerase may also be RNA polymerase obtained by substituting tyrosine for the number 644 amino acid residue phenylalanine and for the number 667 amino acid residue phenylalanine in wild type T7 RNA polymerase. Still further, it may also be RNA polymerase obtained by further substituting proline for the number 665 amino acid residue leucine in wild type T7 RNA polymerase.

Examples of the mutant RNA polymerase includes:

(1) RNA polymerase that is from T3 phage and has tyrosine at amino acid residue number 645 and/or 668;
(2) RNA polymerase that is from K11 phage and has tyrosine between amino acid residue numbers 663-668 or at amino acid residue number 690.
(3) RNA polymerase that is from SP6 phage and has tyrosine between amino acid residue numbers 633-638 or at amino acid residue number 670.

The term "wild type RNA polymerase" means all naturally existing RNA polymerase. The term "wild type RNA polymerase" further includes wild type polymerase in which an amino group has been substituted, inserted, or deleted other than as a modification for improving incorporation of labeled substrate. That is, RNA polymerase obtained by artificially modifying wild type RNA polymerase for some end other than that set forth above is also covered by the term "wild type RNA polymerase." The above-mentioned amino acid substitution, insertion, or deletion is properly conducted in a manner preserving RNA polymerase activity.

The mutant RNA polymerase may be prepared by methods of preparing nucleic acid molecules coding for RNA polymerase, causing mutation of the nucleic acid molecule so that one or more bases at one or more sites in the nucleotide base sequence are varied, and recovering modified RNA polymerase expressed by the varied nucleic acid molecule. Known methods may be employed to prepare a nucleic acid molecule coding for RNA polymerase, introducing mutation into the nucleic acid molecule, and recovering the modified RNA polymerase.

For example, mutant T7 RNA polymerase may be constructed by the following method. Employing a template in the form of an expression vector into which the T7 RNA polymerase gene has been inserted, an expression vector into which mutation has been introduced by the PCR method into the region located between restriction enzyme HpaI and NcoI sites corresponding to the C terminal sides of the T7 RNA polymerase gene is constructed. Next, this expression plasmid is used to transform E. coli DH5 $\alpha$. When isopropyl-$\beta$-D-thiogalactopyranoside (IPTG) is added, large quantities of mutant T7 RNA polymerase protein can be expressed.

(Preparation of RNA probe)

Labeled RNA probe employing mutant RNA polymerase is prepared from the above-described labeled substrate and unlabeled substrate by enzymatically synthesizing nucleic acid transcriptase employing a DNA fragment comprising the promoter sequence for the above-described mutant RNA polymerase as template.

For example, cDNA is synthesized by reverse transcription reaction from mRNA using oligo-dT primer having an RNA polymerase promoter site at the 5' end, and then double-stranded cDNA is prepared by DNA polymerase reaction. The DNA obtained is employed as template, and mutant type T7 RNA polymerase (for example, polymerase in which tyrosine has been substituted for the phenylalanine at 644) is employed to incorporate cyanine 3-UTP or cyanine 5-UTP (labeled substrate) other than the usual NTP substrate. This yields an RNA product comprising cyanine 3-UTP or cyanine 5-UTP. However, the label NTP is not limited to label UTP, there being cases where label ATP, label GTP, and label CTP are employed. The synthesis reactions based on these RNA polymerases may be conducted in the manner set forth above. It is also possible to employ two or more label NTPs (of identical label type) as substrate in a single synthesis reaction with RNA polymerase. This improves the density of the labels present in the RNA probe.

The labeled RNA probe synthesized with the mutant RNA polymerase by the method of the present invention may be employed as is for hybridization with the targeted nucleic acid. Alternatively, the labeled RNA probe synthesized with RNA polymerase by the method of the present invention may be first fragmented (severing the strand into short sections) and then employed in hybridization with the targeted nucleic acid. The labeled RNA probe can be fragmented by heating (for example, to 60° for 30 min) in the presence of a bivalent metallic ion such as $Zn^{2+}$.

Method of Detecting Targeted Nucleic Acid

The method of detecting targeted nucleic acid of the present invention is characterized in that labeled nucleic acid and labeled RNA probe prepared by the above-described method of the present invention are mixed together and RNA probe that has hybridized with the targeted nucleic acid is selectively detected.

The conditions of hybridization of the RNA probe and targeted nucleic acid may be suitably determined based on the type of targeted nucleic acid and the type of RNA probe. For example, hybridization solution comprising the RNA probe may be applied dropwise to targeted nucleic acid that has been fixed to an oligonucleotide array or cDNA microarray and allowed to remain for a prescribed period.

Following mixing and hybridization, RNase processing is conducted, and the remaining targeted nucleic acid and the hybrid with RNA probe are detected to perform the above-mentioned selective detection.

In the RNase processing of the mixture, following hybridization, the oligonucleotide array or cDNA microarray in which the targeted nucleic acid has been fixed is processed with an RNase solution in a suitable buffer.

Hybrids of nucleic acid and RNA probe may be suitably detected by known methods based on the type of label present in the RNA probe.

The targeted nucleic acid may be, for example, DNA, peptide nucleic acid, RNA, or RNA. The targeted nucleic acid may be fixed to a substrate. For example, the targeted nucleic acid may be in the form of a chip or microarray. The substrate to which the targeted nucleic acid is fixed need only be insoluble in the solution; examples are plates, beads, fibers, gels, films, and ceramics. More specific examples are oligonucleotide arrays formed by synthesizing at high density oligonucleotides on a substrate, called DNA chips, and cDNA microarrays in which cDNA amplified by PCR is fixed on a substrate.

For example, in the preparation of a DNA microarray, a PCR reaction is conducted with labeled nucleic acid (for example, the plasmid DNA of individual clones of a mouse cDNA library) as template and the PCR product obtained is fixed to a glass slide coated with poly-L-lysine. Peptide nucleic acid and RNA microarrays may also be prepared in a manner similar to that of DNA microarrays.

Generally, in the case of oligonucleotide arrays, the uniformity and reproducibility of the quantity of oligonucleotide fixed to the substrate are high. In the detection of targeted DNA by probe, data of a certain degree of reproducibility can be obtained using one type of labeled probe.

However, with cDNA microarrays, there are differences in the population of various cDNA contained in the cDNA library and it is impossible to quantify the amount of cDNA from the intensity of the fluorescence or the like from the labeled probe that has hybridized with the targeted DNA. Accordingly, in the case of a cDNA microarray, it is desirable to employ a double-fluorescence labeling method employed fluorescent-labeled probes of two colors to detect labeled DNA with probe.

Labeled DNA may be detected with probe by the usual methods.

For example, for a DNA microarray prepared with the plasmid DNA of the various clones in a mouse cDNA library as targeted nucleic acids, RNA probe labeled with cyanine 3 derived from mouse head mRNA extracted 10 days following birth and RNA probe labeled with cyanine 5 derived from mRNA extracted from 17.5-day mouse embryo are mixed in equal quantities and the signal of targeted DNA on the microarray is detected. In this case, the RNA probe labeled with cyanine 5 derived from mRNA extracted from 17.5 day mouse embryo may be employed as reference to determine the relation (qualitative and quantitative) between the individual cDNAs on the DNA microarray and mouse head mRNA on day 10 after birth.

Kit for Preparing RNA Probe

The kit for preparing RNA probe of the present invention is a kit for preparing labeled RNA comprising:

(1) RNA polymerase;
(2) DNA comprising a promoter sequence for the RNA polymerase;
(3) substrates of the RNA polymerase; and
(4) optionally an instruction manual.

It is characterized in that at least one of the substrates is labeled and in that the RNA polymerase is mutant RNA polymerase in which at least one of the amino acids of wild type RNA polymerase has been modified to incorporate the labeled substrate.

The labeled substrate and mutant RNA polymerase are as follows.

The DNA comprising a promoter sequence for mutant RNA polymerase is DNA comprising a promoter sequence for the RNA polymerase contained in the above-described kit. Since the mutant RNA polymerase is, for example, RNA polymerase from T7 phage, T3 phage, SP6 phage, or K11 phage, DNA comprising the promoter for any of these RNA polymerases is employed.

The kit of the present invention may further comprise a means of linking the above-described DNA comprising the promoter sequence and DNA used for preparation of RNA probe. The means of linking the DNA comprising the promoter sequence and the DNA used for preparation of RNA probe may be, for example, DNA polymerase or DNA polymerase and reverse transcriptase. When employing DNA polymerase or reverse transcriptase as the means of linking DNAs, DNA for preparing RNA probe comprising the promoter sequence can be obtained by synthesizing DNA or RNA using the DNA comprising the promoter sequence as primer.

The kit of the present invention comprises ribonucleoside 5' triphosphates (referred to as NTP derivatives) comprising ATP, GTP, CTP, and UTP, or their derivatives as substrates of the RNA polymerase. All four of these NTP derivatives are preferably included. However, in consideration of combination with labeled NTP derivatives, it is possible to omit NTP derivatives having bases corresponding to the labeled NTP derivatives. In addition to the above-described NTP derivatives, the kit of the present invention comprises at least one type of NTP derivative that is partially or completely labeled. Partially labeled NTP derivatives refer to a mixture of labeled NTP derivatives and unlabeled NTP derivatives. In that case, the mixing ratio of labeled NTP derivatives and unlabeled NTP derivatives is suitably determined in consideration of the amount of label carrier on the RNA probe obtained. Completely labeled NTP derivative means that all of the NTP derivatives are labeled. The kit of the present invention preferably contains 2-4 types of partially or completely labeled NTP derivatives. Including at least two types of partially or completely labeled NTP derivatives makes it possible to prepare two types of RNA probe having different labels. Specific examples of the labels are the same as those described for the method of preparing RNA probe above.

The following combinations of substrates contained in the kit may be used. However, the kit is not limited thereto.

(1) ATP, GTP, CTP, and UTP (unlabeled NTPs) and identically labeled ATP, GTP, CTP, and UTP (labeled NTPs).

(1) above is a kit for preparing RNA probe having a single label. In (1) above, the labeled NTPs may be of one or more types. Cyanine 3-UTP and cyanine 3-ATP are examples of a case where there are two types of labeled NTPs. Unlabeled NTPs comprising the same ribonucleotide as the labeled NTPs may be incorporated or may be omitted. Further, quantities of the individual substrates required for a single polymerase reaction in a single reaction vessel (test tube) may be included, or they may be included in separate vessels and quantities weighed out according to the instruction manual for use.

(2) ATP, GTP, CTP, and UTP (unlabeled NTPs) and ATP, GTP, CTP, and UTP (labeled NTPs) having different labels

(2) above is a kit for preparing two or more RNA probes having difference labels. In (2) above, the labeled NTPs include two or more types of NTPs having different labels. In this case, although the labels are different, there may be a single type of ribonucleotide. For example, the labeled NTPs may be cyanine 3-UTP and cyanine 5-UTP. Unlabeled NTPs comprising the same ribonucleotide as the labeled NTPs may also be incorporated. Further, quantities of the individual substrates required for a single polymerase reaction in a single reaction vessel (test tube) may be included, or they may be included in separate vessels and quantities weighed out according to the instruction manual for use. However, labeled NTPs comprising two or more labels must be contained in separate vessels for preparation of RNA probe having a single label.

**[Examples]**

The present invention is described more in detail in the following examples.

Example 1

1) The effect of mutant RNA polymerase on the incorporation of cyanine 3-UTP or cyanine 5-UTP into RNA

An experiment comparing the incorporation into RNA of cyanine 3-UTP or cyanine 5-UTP by means of mutant RNA polymerase to incorporation by conventional RNA polymerase was conducted in the following manner.

| Template DNA*** (0.1 µg/mL) | 1 µL |
|---|---|

***) The template DNA employed was Riken cDNA clone (GAPDH (glyceraldehyde-3-phosphate dehydrogenase) Riken clone ID 3000002C10).

(continued)

| | |
|---|---|
| 5X buffer solution | 4 µL |
| BSA(2 mg/mL) | 0.8 µL |
| 10 mM ATP | 1 µL |
| 10 mM GTP | 1 µL |
| 10 mM CTP | 1 µL |
| 2 mM UTP** | 1-5 µL |
| 2 mM cyanine 3-UTP (or cyanine 5-UTP)** | 0-4 mL |
| T7 RNA polymerase **** (200 units/µL) | 0.5 µL |
| 0.1 M DTT | 2 µL |
| Water | 3.6-7.7 µL |
| Total | 20 µL |

*) 0.2 M Tris-HCL (pH 8.0), 40 mM $MgCl_2$, 1 mM spermidine-3 (HCl), 125 mM NaCl **) The molar ratios of normal UTP without fluorescent labeling to cyanine 3-UTP (or cyanine 5-UTP) were 1:2, 1:1, 2:1, and 4:1. The UTP concentration of the two was kept constant (maximum concentration 0.5 mM).

****) F644Y (Japanese Patent Un-examined Publication No. Heisei 1175867) was employed as the mutant RNA polymerase.

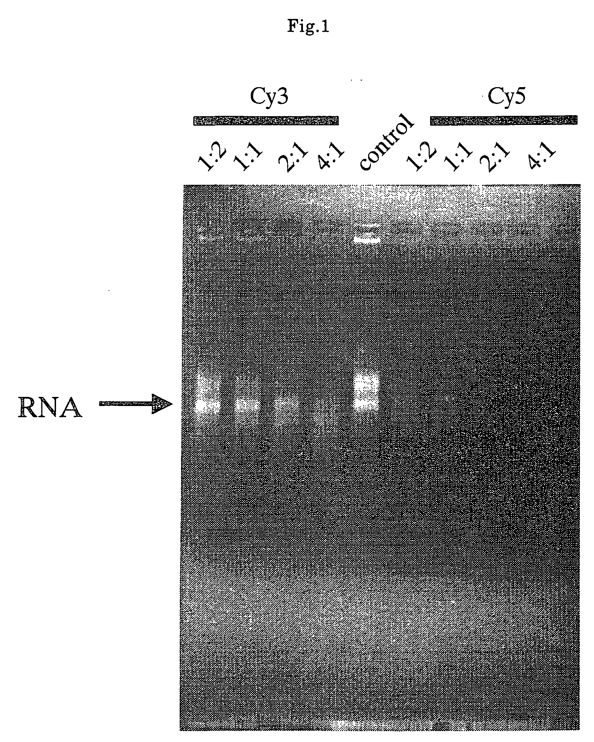

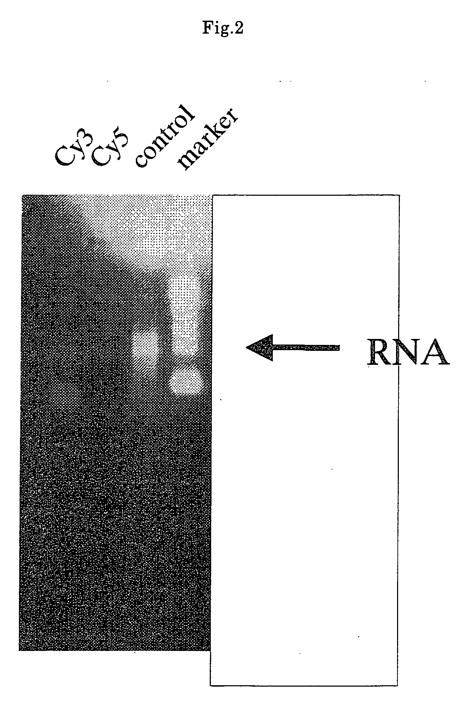

Following reaction for 1 h at 37°C and a decomposition treatment for 10 min at 70°C, the reaction product was isolated on a Clonentech CHROMA SPIN-30 column, and a 2 µL portion thereof was analyzed by electrophoresis (conditions: migration in 16% v/v formamide/1% agarose gel). Fig. 1 shows the results of electrophoresis when the gel was dyed with EtBr following electrophoresis. Figs. 1 and 2 respectively show the results when mutant and wild type RNA polymerases were employed. The remaining product that was recovered was diluted 20-fold and measured with a Beckman DU-600 at wavelengths of 260 µm, 550 µm, and 650 µm, and RNA, cyanine 3, and cyanine 5 were quantitatively determined. The results are given in Table 1. The concentration of cyanine 3 (Cy3) and cyanine 5 (Cy5) in Fig. 2 was made 0.17 mM (corresponding to a ratio of cyanine-UTP to unlabeled UTP of 1:2).

Table 1

| Sample | RNA (A260) | Cy3 (A550) | Cy5 (A650-) |
|---|---|---|---|
| Mutant RNA polymerase | | | |
| Control* | 0.0847 | 0.0016 | 0.0016 |
| Cy3 1:2** | 0.0600 | 0.0105 | 0.0012 |
| Cy3 1:1 | 0.0552 | 0.0166 | 0.0010 |
| Cy3 2:1 | 0.0517 | 0.0236 | 0.0006 |
| Cy3 4:1 | 0.0504 | 0.0335 | 0.0015 |
| Cy5 1:2*** | 0.0626 | 0.0023 | 0.0163 |
| Cy5 1:1 | 0.0580 | 0.0064 | 0.0253 |
| Cy5 2:1 | 0.0504 | 0.0084 | 0.0309 |
| Cy5 4:1 | 0.0279 | 0.0037 | 0.0175 |
| Blank**** | -0.0003 | 0.0005 | 0.0006 |

*) Did not comprise cyanine 3-UTP or cyanine 5-UTP.

**) Indicates that the addition ratio (molar ratio) of cyanine 3-UTP to UTP was 1:2.

***) Indicates that the addition ratio (molar ratio) of cyanine 5-UTP to UTP was 1:2.

****) Indicates absorbance of water alone.

The results of Table 1 indicate that for mutant RNA polymerase, in the synthesis of RNA incorporating cyanine 3-UTP or cyanine 5-UTP, although RNA synthesis was impeded as the concentration of cyanine increased, cyanine was incorporated and RNA synthesis was impeded almost not at all up to a concentration of about twice that of unlabeled UTP. By contrast, in the case of wild type RNA polymerase, as is also clear from Fig. 2, marked impeding of RNA synthesis was observed even at cyanine 3-UTP or cyanine 5-UTP addition ratios relative to unlabeled UTP of 1:2.

Example 2

The effect of fluorescent RNA probe prepared with mutant RNA polymerase on DNA microarray detection

(a) Preparation of target DNA: Using the various cloned DNA of a murine full-length strand cDNA library (see 1-3) comprising cloned DNA previously obtained by the inventors in the laboratory as template, the target DNA was obtained by PCR employing vector-specific primer. The composition of 100 μL of reaction solution was as follows:

| 10xExTaq buffer solution | 10 μL |
|---|---|
| 2.5 mM dNTP Mix | 10 μL (maximum concentration 250 μM) |
| Primer (forward) (10 mM) | 2 μL (maximum concentration 0.2 μM) |
| Primer (reverse) (10 mM) | 2 μL (maximum concentration 0.2 μM) |
| Template DNA | 3 μL (about 10 ng) |
| Water | 73 μL |
| Total | 100 μL |

Examples of primer (forward and reverse) employed are M13 primer (forward) F1224 (5'-CGCCAGGGTTTTCCCAGTCACGA-3') (SEQ ID NO:1) and M13 primer (reverse) R1233 (5'-AGCGGATAACAATTTCACACAGGA-3') (SEQ ID NO:2).

To this were added Ex Taq 1.25 μL (6.25 units in 1 x Ex Taq buffer) and PCR was conducted (3 min at 95°C -> 1 min at 95°C/30 sec at 60°C/3 min at 72°C repeated 30 times -> 3 min at 72°C). The PCR product was confirmed (amplification and contamination check) by agarose gel electrophoresis with part of the reaction solution. The PCR product was then refined, concentrated, and dissolved in 3 x SSC (see References 1 and 2, and Reference 3, Chapter 3).

(b) Preparation of microarray:

Using a DNA arrayer, a glass slide coated with poly-L-lysine was fixed with the PCR product obtained in (a) (see References 1 and 2, and Reference 3, Chapter 4). The usual spot diameter was 100 μm, with 21,168 cDNA spots per slide.

(c) Preparation of probe RNA

Among the conditions described in Example 1, the ratio (molar ratio) of cyanine-UTP and unlabeled UTP was 1: 2 and cDNA obtained by transcription of mRNA prepared from mouse tissue was employed as template DNA. Mutant RNA polymerase was employed to synthesize RNA, which was refined and recovered by the same operations as in Example 1. For comparison with conventional methods, DNA probe was also prepared according to Reference 1. That is, mRNA extracted from tissue was employed as template and a reverse transcription reaction was conducted with random primer to prepare cDNA incorporating cyanine 3-dUTP and cyanine 5-dUTP.

cDNA obtained by reverse transcription of mRNA prepared from the head of a 10-day-old mouse was employed for cyanine 3 labeling, and the promoter sequence of T7 RNA polymerase was inserted into the reverse transcription promoter. cDNA obtained by reverse transcription of mRNA prepared from a 17.5-day mouse embryo was employed for cyanine 5 labeling, and the promoter sequence of T7 RNA polymerase was inserted into the reverse transcriptase promoter.

(d) Hybridization and signal detection:

A hybridization solution in which cyanine 3 and cyanine 5 probe had been combined (ratio by volume: 1:1) was heat treated for 5 min at 70°C (1 min at 95°C for DNA probe) and cooled to room temperature. A 30 μL quantity was then placed on a glass slide with a glass cover. An operation was conducted to prevent drying, hybridization was conducted in a hybrichamber, and the signal was detected with a scanner (see References 3, 7, and 8).

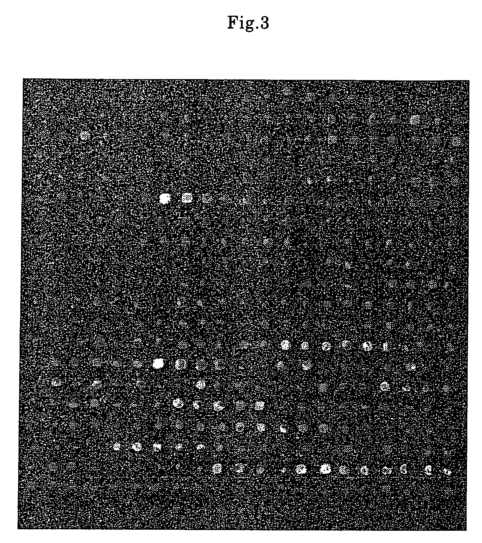

When RNA probe was employed, RNase treatment (4 μg of RNase was added to wash buffer III (0.2 x SSC) and reacted for 10 min at 37°C) was conducted. The microarray pattern of the result following stabilization is given in Fig. 3. However, Fig. 3 only shows one block of a 16-block microarray.

When DNA was employed as probe in place of RNA probe, a clear signal was obtained in some cases, but consistently obtaining clear signals proved problematic.

That is, when RNA probe was employed, it was possible to reduce noise by RNase treatment, and since the stringency of RNA/DNA was greater than that of DNA/DNA, it was possible to achieve a signal with a high S/N ratio. A more consistently stable, clear signal was obtained than with DNA probe.

References

(1) Miki, R. et al. Proc. Natl. Acad. Sci. USA. 98 (2001) 2199-2204.

(2) Miki, R. et al., Cell Technology, Vol. 18, 877-885 (1999).

(3) A DNA Microarray Practice Manual (ed. by Hayashizaki, Y., comp. by Okasaki Y., Yodo Pub., 2000)

**Claims**

**1.** A method of preparing labeled RNA probe by reacting RNA polymerase in the presence of a DNA fragment comprising a promoter sequence for said RNA polymerase and substrates of said RNA polymerase, **characterized in that**

at least one of said substrates comprises said label, and

said RNA polymerase is mutant RNA polymerase where at least one of the amino acids of wild type RNA polymerase has been modified to permit incorporation of the substrate having a label or to improve the incorporation of the substrate having a label.

2. The method according to claim 1, wherein said substrates are ribonucleotide 5' triphosphates comprising ATP, GTP, CTP, and UTP, or derivatives thereof (referred to hereinafter as NTP derivatives), and part or all of one or more of these NTP derivatives comprises said label.

3. The method according to claim 1 or 2, wherein said label is a fluorescent label.

4. The method according to claim 3, where said fluorescent label is cyanine 3 or cyanine 5.

5. The method according to any of claims 1-4, wherein said mutant RNA polymerase is RNA polymerase obtained by substitution, insertion, or deletion of at least one amino acid present at a nucleotide bonding site of wild type RNA polymerase.

6. The method according to any of claims 1-4, wherein said mutant RNA polymerase is RNA polymerase obtained by substituting tyrosine for at least one amino acid present at a nucleotide bonding site of wild type RNA polymerase.

7. The method according to claim 6, wherein the amino acid substituted is phenylalanine.

8. The method according to any of claims 4-7, wherein the amino acid present at a nucleotide bonding site is an amino acid in the loop between helix Y and helix Z and/or an amino acid in the loop between helix Z and helix AA.

9. The method according to any of claims 1-8, wherein the mutant RNA polymerase is from T7 phage, T3 phage, SP6 phage, or K11 phage.

10. The method according to any of claims 1-4, wherein the mutant RNA polymerase is wild type RNA polymerase in which at least one of the amino acids in a region corresponding to amino acid residues 641-667 of RNA polymerase from T7 phage is modified.

11. The method according to any of claims 1-4, wherein the mutant RNA polymerase is RNA polymerase from T7 phage having tyrosine as amino acid residue 644 and/or 667.

12. The method according to any of claims 1-4, wherein the mutant RNA polymerase is RNA polymerase in which tyrosine is substituted for the number 644 amino acid residue phenylalanine of wild type T7 RNA polymerase.

13. The method according to any of claims 1-4, wherein the mutant RNA polymerase is RNA polymerase in which tyrosine is substituted for the number 667 amino acid residue phenylalanine of wild type T7 RNA polymerase.

14. The method according to claim 13 or 14, wherein the mutant RNA polymerase is RNA polymerase in which proline is further substituted for the number 665 amino acid residue leucine of wild type T7 RNA polymerase.

15. The method according to claim 1-4, wherein the mutant polymerase is RNA polymerase in which tyrosine is substituted for the number 644 amino acid residue phenylalanine and tyrosine is substituted for the number 667 amino acid residue phenylalanine of wild type T7 RNA polymerase.

16. The method according to claim 15, wherein the mutant RNA polymerase is RNA polymerase in which proline is further substituted for the number 665 amino acid residue leucine of wild type T7 RNA polymerase.

17. The method according to any of claims 1-4, wherein the mutant RNA polymerase is RNA polymerase from T3 phage having tyrosine at the number 645 and/or 668 amino acid residue.

18. The method according to any of claims 1-4, wherein the mutant RNA polymerase is RNA polymerase from K11 phage having tyrosine between the number 663-668 amino acid residues, and/or at the number 690 amino acid residue.

**19.** The method according to any of claims 1-4, wherein the mutant RNA polymerase is RNA polymerase from SP6 phage having tyrosine between the number 633-638 amino acid residues, and/or at the number 670 amino acid residue.

**20.** A method of detecting targeted nucleic acid in which targeted nucleic acid and labeled RNA probe prepared by the method according to any of claims 1-19 are mixed and RNA probe that has hybridized with the targeted nucleic acid is selectively detected.

**21.** The method of detection according to claim 20, wherein following the mixing and hybridization, the mixture is treated with RNase and the remaining targeted nucleic acid and the hybrid with RNA probe are detected to conduct the selective detection.

**22.** The method of detection according to claim 20 or 21, wherein the targeted nucleic acid is fixed to a substrate.

**23.** The method of detection according to any of claims 20-22, wherein the targeted nucleic acid is DNA, peptide nucleic acid, or RNA.

**24.** The method of detection according to any of claims 20-22, wherein said targeted nucleic acid is in the form of an oligonucleotide array or cDNA microarray.

**25.** A kit for preparing labeled RNA probe comprising

    (1) RNA polymerase,

    (2) DNA comprising a promoter sequence for said RNA polymerase, and

    (3) substrates of said RNA polymerase;

    **characterized in that** at least one of said substrates comprises a label and said RNA polymerase is mutant RNA polymerase where at least one of the amino acids of wild type RNA polymerase has been modified to permit incorporation of said substrate having a label or to improve the incorporation of said substrate having a label.

**26.** The kit according to claim 25 further comprising a means of linking the DNA comprising a promoter sequence and the template DNA for preparing probe.

**27.** The kit according to claim 26, wherein said means of linking the DNA comprising a promoter sequence and the template DNA for preparing probe is DNA polymerase, or DNA polymerase and reverse transcriptase.

**28.** The kit according to any of claims 25-27, wherein said substrates comprises all or some from among ribonucleotide 5' triphosphates consisting of ATP, GTP, CTP, and UTP, or derivatives thereof (referred to hereinafter as NTP derivatives), and in addition to said NTP derivatives, at least one NTP derivative all or part of which has labels.

**29.** The kit according to claim 28, wherein said kit comprises two or more NTP derivatives all or part of which have labels.

**30.** The kit according to any of claims 25-29, wherein said label is a fluorescent label.

**31.** The kit according to claim 30, wherein said fluorescent label is cyanine 3 or cyanine 5.

Fig.1

Fig.2

Fig.3

```
                          SEQUENCE LISTING



    <110>   RIKEN
            HAYASHIZAKI, Yoshihide

    <120>   Method for preparation of RNA probe, method for
            detecting targeted nucleic acid, and kit for prepration
            of RNA probe.

    <130>   3561PTEP

    <150>   JP 167910/2001
    <151>   2001-06-04

    <160>   2

    <170>   PatentIn version 3.1

    <210>   1
    <211>   23
    <212>   DNA
    <213>   Bacteriophage M13

    <400>   1

    cgccagggtt ttcccagtca gca                                      23


    <210>   2
    <211>   24
    <212>   DNA
    <213>   Bacteriophage M13

    <400>   2

    agcggataac aatttcacac agga                                     24
```